# EUROPEAN PATENT APPLICATION

(11) **EP 2 894 228 A1**
(43) Date of publication of application: **15.07.2015**
(21) Application number: 12882330.9
(22) Date of filing: 31.07.2012
(51) Int. Cl.: C12Q 1/68, G09F 3/00

(54) **METHOD FOR OBTAINING AND DETECTING A MARKER OF OBJECTS TO BE IDENTIFIED, RELATED MARKER, AUTHENTICATION METHOD AND VERIFICATION METHOD**

(71) Applicant: Jaime, Juan Carlos, Córdova X5000AGC (AR); Simonetta, Rubén, Córdova X5000AGC (AR); Naranjo, Mauricio, Córdova X5000AGC (AR); Lopez, Joaquín, Providencia, Santiago 7500545 (CL)
(72) Inventor: Jaime, Juan Carlos, Córdova X5000AGC (AR); Simonetta, Rubén, Córdova X5000AGC (AR); Naranjo, Mauricio, Córdova X5000AGC (AR); Lopez, Joaquín, Providencia, Santiago 7500545 (CL)
(74) Representative: Tischner, Oliver
(86) International application number: PCT/CL2012/000036
(87) International publication number: WO 2014/019099

(57) **Abstract**

Procedure for obtaining a marker of objects to be identified, comprising at least one polymorphic DNA fragment and preferably a plurality of polymorphic DNA fragments, such as microsatellite or single base polymorphism, modified in their three-dimensional structure, adsorbed to metallic nanoparticles and micro encapsulated, which are active to Raman radiation. Said marker is also protected; as well as the marker detection method; the method to instantly authenticate marked objects with the said marker; the method to verify the marked objects with the said marker and the method for incorporating the said marker to the desired object to be identified.

## Description

### Abstract

The present invention is related to the field of biotechnology, specifically to molecular biology, and refers to a method to obtaining a marker for identifying objects comprising of at least one DNA fragment, and preferably a plurality of polymorphic DNA fragments, such as microsatellites (STRs) or single nucleotide polymorphism (SNPs) among others use for forensic purposes, modified on their three-dimensional structure, adsorbed to metal nanoparticles and microencapsulated that are active to RAMAN radiation. A method for performing detection/instantaneous authentication and subsequent identification by molecular biology techniques, that globally involves a security system comprising ten levels.

The detection method of the genetic marker obtained, the method to instantly authenticate objects marked with the said procedure, are also protected herein; as well as the method to verify the objects marked and the method for incorporating a label to an object to be identified.

The components, to which reference is made, may be selected from various equivalents without implying any deviation from the principles of the invention set forth in this documentation.

### Background of the invention

The attempt to find new provisions or mechanisms to give greater security in routine transactions, is permanently made.

Since ancient times man has tried to prevent theft, deception and forgery, using documents that should be modified to incorporate elements that resulted difficult to reproduce. Sealings with especial figures obtained from different dies, fulfilled the expectations of the early days, but had quickly changed the printing methods; the incorporation of new security features that technical advances and the emerging quality instruments had been overcoming.

When erasing values from bank checks, to redo them with higher values, the marking of paper has opposed.

Counterfeit paper money and documentation in general, has led to the adoption of special papers and inks, optical inks, the incorporation of security elements in the paper matrix, translucent protection films, etc.

The inventors are aware of elements that are specific for each person. Such is the case of fingerprints and such is also the case of DNA.

Indeed, each individual has a biological trace, which is unique and that current techniques can identify with absolute certainty.

While this is true, the inventors clearly know that incorporation of a complete DNA molecule of a living being to an object for accurate positive identification, it would be easily exploited by counterfeiters; since it would be sufficient to be near the owner of such DNA and get a biological sample from it.

Indeed, a hair, saliva deposited on a glass, a drop of blood and even a speck of skin would be sufficient to obtain the DNA required to add to a fake object; then, it will obviously appear to be true.

This has already been foreseen by the inventors, in the document 200020102319 AR, which gave rise to the presentations US 10/307012 and EP 33801366; these are considered the closest to the theme developed in this documentation prior art.

Indeed, considering the possibility stated, the inventors have disclosed in the above documents that the object to be identified should be labeled with at least one specific polymorphic DNA fragment.

By using only one polymorphic fragment of the DNA, it is not enough for the forger to obtain the full DNA, cause he must necessary know which is the chosen fraction (or combination of fractions) of DNA used. Whereas, there are billions of possible combinations, it is almost impossible for forgers to utilize by chance the user-selected combination, thus resulting a highly effective genetic marker.

While the marker covered by the documents cited is highly effective, the inventors have found that the processing power of computers allows millions of operations per second increasing the chances of counterfeiters.

The inventors know that, as disclosed in the cited documents, the detection of the DNA fragments used as marker of objects, requires high tech infrastructure that is not available in all the laboratories; so that a simplification of the detection method of polymorphic DNA fragments would expand the use of this type of markers.

Moreover, as any molecular scientist should recognize, although the laboratory who wants to make counterfeiting may be very specialized in these techniques, such polymorphic fragments detailed herein (STRs / SNPs), cannot be duplicated unless the exact nucleotide sequence is previously known; because the reagents (primers) needed for lab detection, are fabricated based on this knowledge.

With such observations on the disclosed matter, the inventors have determined that it is an objective of this invention to obtain a marker formed from polymorphic DNA fragments of any living being existing in nature.

This implies that the fragments to be used can be obtained from humans, as well as animals, plants, microorganisms or viruses.

To obtain the marker disclosed in this document, it is perfectly convenient the use of a combination of different polymorphic DNA fragments obtained from all these species.

In order to make instant detection by Raman spectroscopy, and to also increase the complexity of the marker, which serve to avert counterfeiting, the inventors have proceeded to geometrically modify the three dimensional structure of such polymorphic fragments, to be adsorbed then on nano metal particles.

This greater complexity prevents the forger to reveal the structure of the marker, especially when it is integrated with relative and absolute concentrations of the STRs and SNPs used.

A second objective of the present invention is, to have a method and apparatus which allow detection and instantaneous authentication, in real time, of the marked objects.

A third object of the present invention is, to have a procedure comprising multiple security levels; which counterfeiters should overpass to reproduce such genetic marker.

Deoxyribonucleic Acid (DNA) is a macromolecule which the genetic information of a particular individual is stored.

The three dimensional geometric structure of DNA is a double helix, one can observe a major groove and lower groove. The first is deep and wide while the second is shallow and narrow.

It is known by the inventors that there are molecules such as proteins, which bind to the inside of the grooves of DNA, by specific binding called hydrogen bonds, and non-specific binding known as interactions of Van der Waals, and other electrostatic interactions .

In the case of proteins, they recognize donors and acceptors of hydrogen bond and methyl groups (hydrophobics), these latter being exclusively for the major groove.

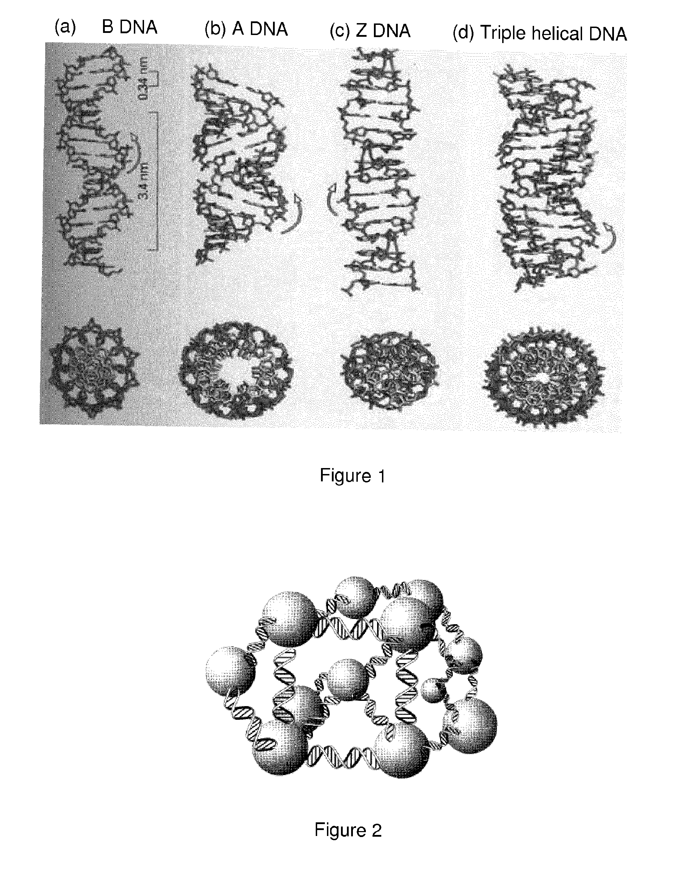

There are four possible recognition patterns in the major groove, and only two in the minor as shown in Figure 1.

We know that some molecules bind to DNA by the major groove while others bind at the minor groove, while some others are joined at both grooves.

In this third case, the event destabilizes the DNA molecule.

All living beings of the same species share many identical pieces of DNA but there are other parts that are different. These different parts are called "polymorphic fragments" which are defined as one of two or more forms (alleles) existing in a specific chromosomal locus that differ in nucleotide sequence or have variable numbers of tandem repeats.

Polymorphic fragments are millions and make every living thing existing in nature as unique.

These polymorphic DNA sites may eventually be used for identification purposes as in forensics.

Some polymorphic DNA fragments exhibit length variations in tandem as minisatellites (VNTRs) and microsatellites (STRs), depending on the number of nucleotides present in the repeatedly core sequence; while others exhibit variations in sequence composition such as a difference in a single nucleotide (SNPs).

Any polymorphic DNA fragment has two allelic variants, each inherited from one parent.

For STRs, allelic variants exist in a number ranging from 7 to 15, on average; while SNPs only have two allelic variants per locus.

SNPs, however, even when they have less discriminatory power than STRs, have the great advantage of being present in the genome amounts in millions; they are responsible for the phenotypic characteristics of living things among ather functions.

In summary, every living being is unique and is therefore different from anyone else on the planet. This diversity in nature depends on the polymorphic sites of DNA, such as microsatellites (STRs) and single base polymorphisms (SNPs).

In the documents to which reference was made and in this, STRs and SNPs are used; firstly because the feasibility to be analyzed and detected by PCR amplification methods, and secondly, because there are millions to select between all living beings that exist in nature.

It has been said that it is an object of the present invention, the instant detection of polymorphic fragments, for which the inventors use the technique of Raman spectroscopy, and preferably, SERS Raman (Surface Enhanced Raman Spectroscopy) which provides one of the most sensitive methods, quantitative and nondestructive material to analyze qualitative analysis. The appliances refer to detect by SERS Raman, a solution of 0.9 nM (nanomolar) of STRs/SNPs, the minimum detection volume 100 -150 fentoliters, containing at least 60 molecules of STRs.

Raman spectrum is similar to an infrared spectrum and consists of a wavelength distribution of bands which correspond to the specific molecular vibration of the sample analyzed.

For this reason, when an emission spectrum Raman is analyzed, the peaks observed corresponding to the wavelength are characteristic of the molecular chemical structure and composition of each analyte; while the intensity of Raman light scattered by molecules in the sample depends on the concentration thereof.

In practice, a Raman spectroscope comprises a light source, usually a laser, which is focused on the sample generating an inelastic scattered radiation, which is optically collected and directed into a spectrophotometer selectively wavelength, wherein the detector converts the energy that print the photons in electric signal intensity. With SERS techniques, Raman signal can be increased 10⁶ a 10¹⁴ fold, making it possible to achieve a sensitivity that allows single molecule detection.

For maximum sensitivity, the adsorption of polymorphic STRs fragments and / or SNPs in nanoparticles of at least one metal selected from gold, silver, platinum and copper is caused, wherein the size of said nanoparticles varies in a range between 5 and 200 nm. This increased sensitivity is achieved because the metal particles form a rough surface on the order of 10 nanometers which is small compared to the wavelength of the incident radiation. The small particle size (optimally between 50 to 100 nm) enables the excitation of the metal and increases the sensitivity of detection of its surface adsorbed DNA.

We proceeded to the certifying of several documents determing that the document number CN1302905 relates to an anti-counterfeit materials containing metal ions DNA prepared by mixing an aqueous solution of a soluble metal salt with high power of coordination with a solution of DNA and alcohol, to obtain an M-DNA decanted water soluble with the addition of gelatin, dextrin, the aqueous solution of a soluble starch or gum for marking or printing ink.

Document No. US2008 / 0189066 relates to the use and modification of a Raman spectroscope to authenticate objects that have been marked with different fragments SNP or STR. For the above mentioned document, it is noted that the fragments detected are obtained without modification; that is, selecting as naturally occurring.

Document No. US20030235836 relates to use of polymorphic STRs fragments or micro encapsulated SNPs that are used for marking objects which are detected in laboratory by molecular biology techniques.

The US 20080189066 patent relates to a method and apparatus for authentication using Raman spectroscopy to authenticate items that have a security mark containing a DNA fragment to prevent fraud using a Raman spectrometer. The peaks in the Raman spectrum are detected to generate data Raman peaks as safety mark. The data security Raman peak is compared to a library of Raman peaks to determine if a match exists.

These techniques allow an instantaneous identification might be called indirect because it is produced by detection of various chemical substances incorporated in the transport medium.

As in the document on Raman spectroscopy modifications, the polymorphic fragments mentioned are obtained without any modification.

### Summary of the Invention

The disclosed invention is a method for obtaining and detecting a genetic marker in the objects to be identified, a method for instant authentication of the marked objects and a verification method for said objects and said marker.

The present invention consist in a marker of objects to be identified which discloses the addition of at least one polymorphic DNA fragment, and preferably the incorporation of a plurality of DNA fragments polymorphic microsatellite type (STR) and single base polymorphism (SNP) modified in 3D molecular structure, absorbed to nanoparticles in the objects to be identified for subsequent detection or instantaneously authentication and identification by molecular biology techniques.

The invention also comprises a method for obtaining said marker, comprising a first step of selecting at least one living being to proceed to the extraction of DNA from any of its cells; a second step of purification of DNA obtained; a third step of amplification of polymorphic microsatellite fragments and / or single base polymorphism; a fourth step of determining the allelic variants of at least one living being selected; a fifth step of modifying the geometric structure of the DNA polymorphic fragment; sixth step of concentration and microencapsulation of DNA; a seventh step of solubilization of the microcapsules containing DNA; eighth step of determining and / or correction of the degree of fluency and concentration of the solution and a ninth step of incorporating the solution by a suitable applicator to the desired object.

The invention also comprises a method for detecting the genetic marker instantly by Raman SERS technology.

Also the invention comprises a method including a first step of instant validation or authentication by comparison of the resulting spectrum to spectras stored in a database, and a second step of identifying polymorphic fragments used in a moelcular biology laboratory using suitable pairs of primers to amplify the fragments STRs or SNPs employed and determining their allelic variants.

### Brief description of the Figures

Figure 1 gives an overview of the different modifications of the three-dimensional structure of DNA.
Figure 2 corresponds to an outline of nano-tag DNA. Bonding metal particles are shown with DNA, forming a true molecular net feasible to be detected by Raman SERS.
Figure 3 corresponds to a schematic representation of DNA bind to dendrimers, which form three-dimensional structures with a specific Raman spectrum.
Figure 4 corresponds to a schematic representation of aptamers (peptides or oligonucleotides) that bind to the DNA molecule forming three-dimensional structures with a specific Raman spectrum.
Figure 5 corresponds to a representation of a DNA intercalating agent such as ethidium bromide, which affect the three dimensional structure of the DNA and cause changes to the Raman spectrum.
Figure 6 corresponds to a Raman spectrum of DNA treated with intercalating agent ethidium bromide.
Figure 7 is a schematic representation of various proteins that bind to the major and minor grooves of the DNA double helix destabilizing its three dimensional structure and generating a specific Raman spectrum.
Figure 8 corresponds to divalent metal-DNA complex, which produce aggregates that alter the Raman spectrum between 1300-1400 cm⁻¹.
Figure 9 corresponds to an emission spectrum Raman, which shows that the peaks corresponding to the wavelength are characteristic of the molecular chemical structure and composition of each analyte, while the intensity of Raman light scattered by molecules in the sample is dependent on the concentration thereof.
Figure 10 corresponds to a diagram of the use of DNA interspecies as Tag anti counterfeiting.

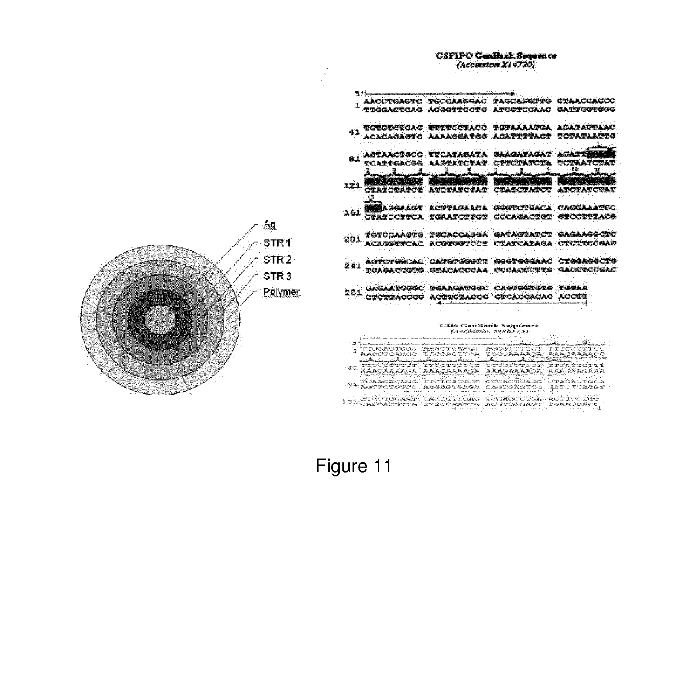

Figure 11 corresponds to a diagram of the use of personal DNA as Tag DNA anti counterfeiting.
Figure 12 corresponds to a diagram of the use of phenotypic SNPs to authenticate passports.
Figure 13A, DNA sample extracted from the saliva of a person and the six SNPs listed genes are amplified.
Figure 13B, shows the possible scenario for determining hypothetical color of blue, brown eyes according to genotypic variants of these six SNPs.
Figure 14 is a representation of the use of microspheres with polymorphic DNA as tag anti-counterfeiting in paper money.

### Detailed description of the preferred embodiments

Having established the components of the invention and the sequence of steps developed to explain their nature, what follows is the description of their functional and operational relationship thereof and the results they provide.

In order to have a marker of objects to be identified that constitutes a secure way to protect valuables, and an instant detection, the present invention promotes a chemical compound that can be used as a marker and a method by which this achieved marker be incorporated to an object, allows the identification and validation of the object.

Preferably, the inventors have found that this marker should be a chemical compound that can be detected later, but only by people who know their structure and using a Raman spectroscope or in the case of ignoring the structure, those using a Raman spectroscope in association with a database structure where polymorphic fragment is stored and encrypted.

Among all the chemical compounds that can be used and based on the explanations disclosed in this document, inventors prefer to use the deoxyribonucleic acid (DNA) as a marker to identify objects.

Preferably said marker to identify objects, consist of at least one polymorphic DNA fragment.

Regarding the method of incorporating the marker consisting in at least one polymorphic DNA fragment to identify the objects, the present invention comprises a plurality of steps where in a first step proceeds to select at least one living being to perform DNA extraction that will be used further on.

The use as a marker of at least one polymorphic DNA fragment of a living being advocated by the inventors, should be interpreted in a broad sense. This means that the decision-maker to perform the marking of an object, can select itself as donor of the DNA fragments or may select any living being, whether animal or plant; so this will further reduce the possibility of falsifying the marker.

DNA extraction is performed from cells or body fluid obtained by common techniques, such as buccal swabs, blood puncture, epithelial collecting samples, hair follicles, etc.

In a second step DNA is released from nucleated cells, in a solution containing 10 mM Tris-HCl -. 0.1 mM EDTA, 20% SDS (w/v) and Proteinase K 10 mgr/ml, and subsequent purification with phenol/chloroform - 10: 9 (v/v).

In a third step STRs and/or SNPs are amplified by the Polymerase chain reaction as recommended in US Patent Nos 4683195; 4683202 and 4800159. The mixture is placed in a thermocycler, containing the DNA sample at a concentration of between 6 and 0.05 pgr; 10X PCR buffer solution, 10X dNTP, 10X oligonucleotides flanking the polymorphic region and Taq polymerase 5,000 units per ml.

In a fourth step, allelic variants of the selected polymorphic fragments are typified in an automatic DNA sequencer ABI PRISM 310 (Applied Biosystem) or similar.

In a fifth step and as the DNA molecules have a unique Raman spectrum because they are made of the same four nucleotides, we proceed to modify the 3 dimensional structure or the polymorphic fragments STRs or SNPs. Subsequent adsorption to nanoparticles of at least one metal selected from gold, silver, platinum and copper; while they are formed from the reduction of its cations according to the method of Lee and Meisel (J. PHYS CHEM 86..: 3391- 3395, 1982): metal nanoparticles have a size of between 5 and 200 nm.

The three dimensional structure of the DNA can be modified from three base structures known in nature A-DNA, B-DNA and Z-DNA; and other possible conformations as C-DNA, DNA-D, E-DNA, L -DNA, P-DNA, S-DNA, etc., as well as the H- DNA triple chain, G4-DNA, or quadruple DNA. Notably, many of the DNA conformations are due to the amount of GC having on the DNA sequence, this characteristic is fully used in this development, resulting on a spectrographic Raman signature, unique and specific for the polymorphic fragments; thus adding an additional level to the identification of polymorphic STRs or SNPs fragments and preventing its reproduction by a possible counterfeiter.

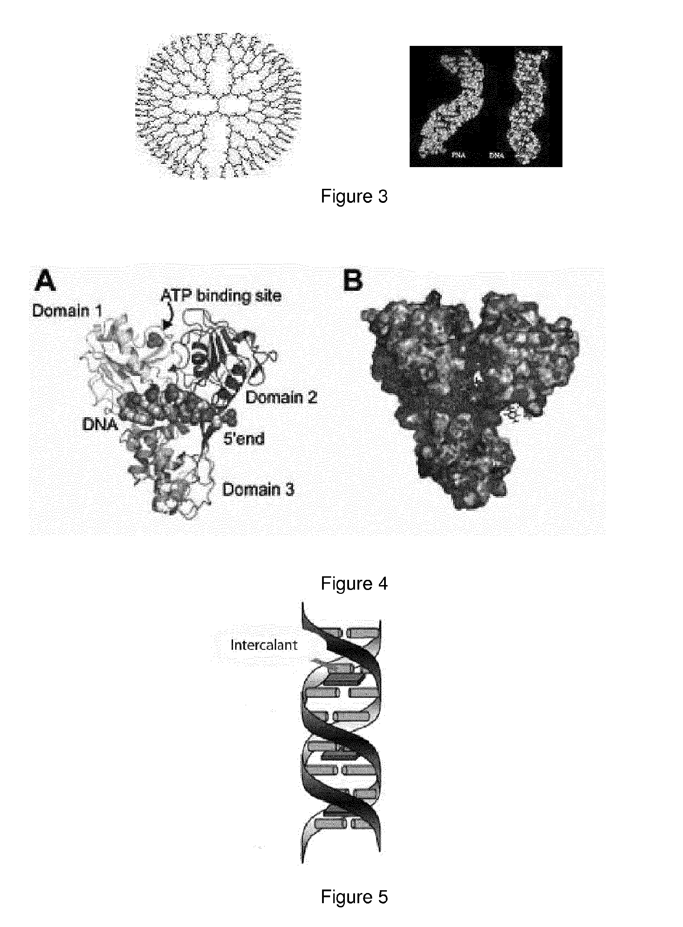

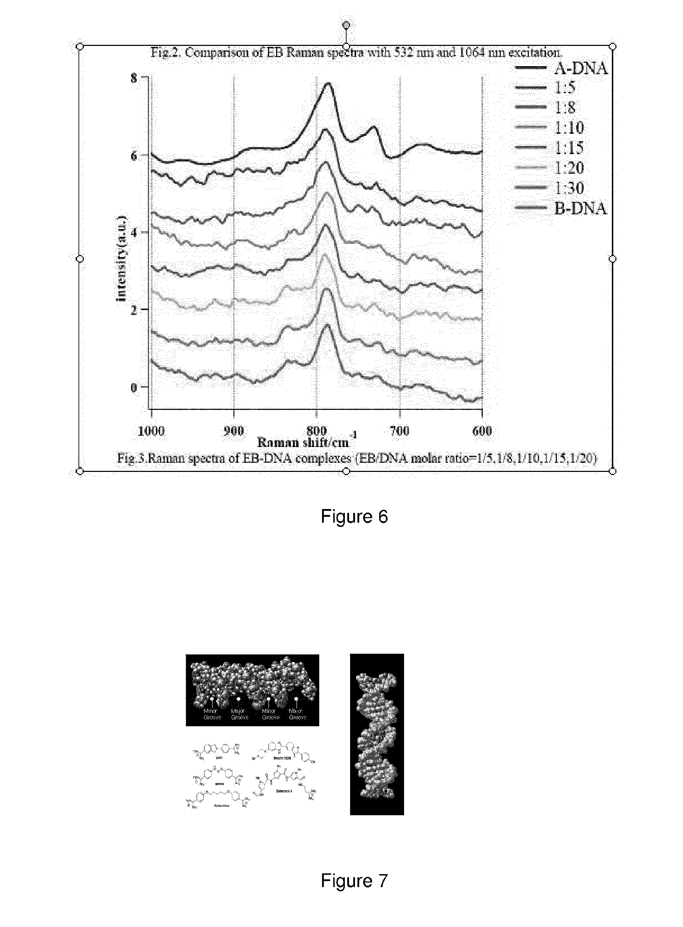

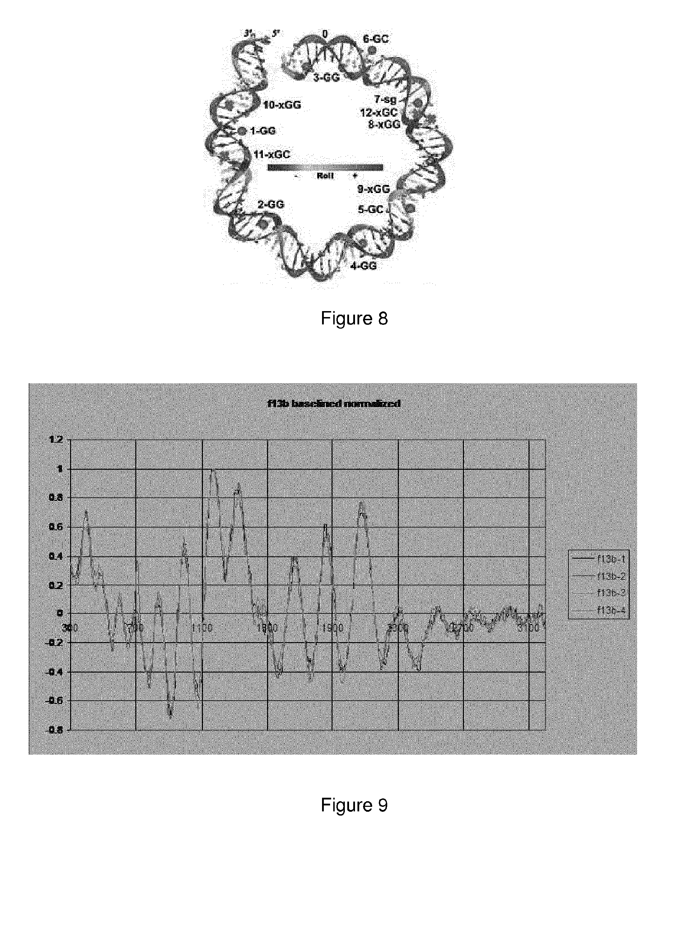

The inventors know that there are multiple ways to modify the three-dimensional structure of DNA so the procedure described was carried out using one of the following methods:
a) Metal nanoparticles with the adsorbed DNA that have been incorporated as the marker, can enhance the Raman SERS signal between 10⁶ and 10¹⁴ and 1014 times and, can be added individually or in the colloidal form, producing aggregates as dimers, trimers, tetramers, etc. together with the DNA molecules. This property is even enhanced when at least a linking group such as a polymer selected from phenylacetylene, polytetrafluoroethylene, polypropylene, polyacrylamide, etc is added to the metal nanoparticles; as shown in Figure 2.
   The linking group may also be selected from other types of molecules such as silanes, alkanes or their derivatives that bind to the DNA molecules, forming specific aggregates as a genuine molecular network which is detected as a unique Raman spectrographic signal.
b) The structure can be also modified with DNA-dendrimers complex, such as PAMAM dendrimers or similar, type forming a three-dimensional structure characteristic resulting in a unique Raman spectrum as described by Caminade AM, in "Characterization of dendrimers", Advanced Drug Delivery Reviews, 57 , 2130-2146, 2005; and as shown in Figure 3.
c) Another way to achieve the 3D modification is through the covalent binding of the polymorphic DNA fragments with synthetic DNA PNAs (peptide nucleic acid). Indeed, the PNAs are a type of polyamide that are analogous to DNA analog with monomeric units for adenine, guanine, thiamine and cytosine where sugar bonds corresponding to an oligonucleotide are replaced by amide bonds. PNAs compounds were discovered by Nielsen (Science, 254: 1497-1415, 1991) and can be acquired from companies such as PE Biosystems (Foster City, California); as shown in Figure 4.
d) Another linking group which can be used, consists of the union of polymorphic fragments to "aptamers", which are a kind of DNA created through repetitive cycles of in vitro selection. The process called SELEX (systematic evolution of ligands by exponential enrichment) involves producing repeated cycles of exposure of a potential aptamer (ligand DNA) to the polymorphic STR or SNP fragment, allowing binding to occur and then, separating the DNA free from ligands and simplify them to repeat the bonding process. After a number of cycles, aptamers exhibit high affinity and specificity, and preserving DNA against the polymorphic fragments used as target. Since aptamers are comprised of oligonucleotides, they can be easily incorporated into fragments STRs or SNPs; as represented in Figure 5.
   For this case, the methods of preparation of aptamers are well known in 5.270163 US patents, US.5.567588, US.5.670637 and US.5.843653.
e) Intercalating agents fit between the bases along the DNA molecules so that such interspersed agents affect DNA structure, producing changes in the torque of the double helix at the intercalation site; which in most intercalating agents results in a GC sequence.
   As an example we can cite the following intercalators ethidium bromide, adriamicyn, 9-aminoacridine (9AA) and proflavine (PF) (3,6-diaminoacridine), well described by James M. Benevides in "Mechanisms of drugs - DNA recognition distinguished by Raman spectroscopy "Journal of Raman Spectroscopy-Vol 39 Pag 1627-1624 among others.; as depicted in Figure 6.
f) Another group of union that allows the linking of the protein with the major and minor grooves of DNA, mainly used drugs that interact with both grooves; so when a precipitation or aggregation occurs there is a change in the three dimensional structure of DNA. Some examples of these kind of drugs are Spermine that interacts with the major groove and spermidine, putrescine, Hoechst 33258, Netropsin, Pentamidine, etc. that interacts with the minor groove.
   A detailed description of each drug that interacts with the major and minor grooves of DNA, can be seen in "NBD Atlax NMR drug index - DNA complexes". The interaction of these drugs with the major and minor grooves of the DNA is directly related to the amount of AT, thus the sequence of each SNP or STR fraction can be obtained by a characteristic spectrum of polymorphic fragment used to label the object; as depicted in Figure 7.
g) There exist DNA-metal divalent complex, such as Sr⁺², Ba⁺², Mg⁺², Ca⁺², Mn⁺², Co⁺², Ni⁺² y Cd⁺², form aggregates that produce variations between 1300-1400cm⁻¹; because they react with phosphate and/or with nitrogen bases by destabilizing the double helix breaking hydrogen bonds, as described by JG Duguid at work "Raman Spectroscopy of DNA-Metal Complexes" (Biophisical Journal Volume 69 Dic.1995- Pag 2623 to 2641, as represented in Figure 8.

In the sixth step, we proceed to concentrated by ultracentrifugation for which the type of Centricon 100 microconcentrators are used; then and to avoid degradation, polymorphic fragments by phase inversion technique are microencapsulated. Microencapsulated polymorphic DNA is dissolved in a solvent, and then in the same solvent, polymer is dissolved in a concentration of 0.25% and 10% w/v. The polymer used can be selected, indifferently among those biodegradable and non-biodegradable.

Within the first, we preferred those such as lactic and glycolic acids and esters such as polyanhydrides, polyurethanes, butyric polyacid, the polyacid Valerino, etc.

Meanwhile, within the non-biodegradable polymers, we preferred the use of ethylene vinyl acetate and polyacrylic acid, resulting also acceptable to use polyamides and copolymers and mixtures thereof.

We can also use polymers selected from natural, in this case it is preferable to employ at least one from the group comprising dextran, cellulose, collagen, albumin, casein, or similar.

The resulting mixture is subsequently introduced into a non-solvent, in a solvent/non-solvent ratio of at least 1/40 to 4/200, for the spontaneous formation of microcapsules. In this step, the solvent is an organic solvent selected from chloroform and methylene chloride, while preferable non solvents are ethanol and hexane.

Additionally, we can incorporate other DNA fragments than those chosen to mark the object, in order to mask the polymorphic DNA fragments selected; and make it even harder to fake.

In a seventh step we proceed to solubilize the DNA microspheres or the microencapsulated DNA in a solution, which is neutral to Raman spectroscopy detection.

In order to mask the Raman spectrum corresponding to the polymorphic DNA fragments selected, and making the marker more difficult to reproduce; we can use a mixture of Raman active organic substances, but with no influence or alteration effect to the spectrum emitted by the polymorphic fragments; Obtaining theoretically millions of specific spectras in a emission range between 500 and 2000 cm⁻¹. These substances are well described in US patent 20060234248.

The microspheres containing the polymorphic fragments may be solubilized in different varieties of ink, such as flexographic ink, lithographic ink, screen ink, gravure ink, currency reactive ink, erasable ink, pen reactive ink, heat reaction ink, visible to infrared ink, optimally variable inks, penetrating inks, photochromic inks, chemically reactive ink to solvent or water.

In an eighth step, we proceed to determine, and correct if necessary, the degree of fluency and concentration of the solution, to enable its appropriate application to the objects to be marked.

It has been estimated that the degree of fluidity of the solution in an applicator, should have a concentration of between 6 and 10 pg of pg per mm^{2.} of marking surface.

Finally, in the ninth step, the marker is carried within an applicator that can be selected from a pen to pen, microfiber, pen, various types of filters, atomizer, drawing tool, brush, stamp or an automatic machine as embodied an electrophotographic printer or inkjet type machine offset lithography, letterpress, gravure, electrophotography, screen printing systems and printing textiles, etc.

In an alternative embodiment, a means intermediary between the solution containing the marker and the object to be marked is emplyed; in which case, said intermediary means is embedded in said solution.

The intermediary means may be selected from various substances, such as nitrocellulose, paper, wood, cardboard, plastic, reinforced nylon, cloth, organic substances as droplets or gel, inorganic, etc.

After selecting the applicator, we proceed to the marking of the desired objects.

The invention also comprises a method of detecting the label, through the detection of the polymorphic DNA fragments included in the genetic marker applied, by a suitable method for detection nucleotides, including but not limited to: Normal Raman scattering, Resonance Raman scattering, surface enhanced Raman scattering, surface enhanced resonance Raman scattering, coherent anti-Stokes Raman spectroscopy (CARS), stimulated Raman scattering, inverse Raman spectroscopy, Raman spectroscopy stimulated gain, hyper-Raman scattering, molecular optical laser examiner (MOLE) or Raman microprobe or Raman microscopy or confocal Raman microspectrometry, three-dimensional or scanning Raman, NIR spectroscopy Raman spectroscopy saturation, time resolved resonance Raman spectroscopy,, decoupling Raman or UV-Raman microscopy.

The Raman spectrometer used to generate the spectrum of polymorphic fragment present in the marked item, can be a desktop or portable device, with a laser wavelength within the range of 400-1200 nm, with variable voltage. In the present invention we have used portable Raman detectors with laser 633 and 785 nm, but this is not limited to devices with other features; as shown in Figure 9.

The invention also comprises a method for validation, which comprises a **first step** of suppressing the characteristic fluorescence of the DNA; a second **step** of comparing the upper and lower values of the intensity of the peaks obtained in the spectrum emitted by the polymorphic DNA with limit reference values previously stored in a library of spectra, resulting on an instant response either by positive or negative authentication.

In reference with the said first stage, and with the operational features of the spectrograph Raman used, the suppression of fluorescence is achieved with a series of steps comprising a step of determining an average intensity value in the spectral data obtained in a section around each point of the response spectrum, and a step of subtracting said average value from each of the points of the spectrum corresponding to the DNA marker.

Meanwhile said second step comprises a step of comparing the data of the Raman peaks obtained, with the Raman peaks data stored in the database; and a step of comparison of the wave numbers and intensity of each peak with the spectrographic data stored in the aforementioned database.

Said stored data correspond to the spectrum of each of the polymorphic fragments STRs or SNPs that have been used to mark the object.

The inventors know that the wavelengths in an emission Raman spectrum are characteristic of the chemical composition and structure of the molecules in a sample, while the intensity of the scattered light is dependent on the concentration of molecules in the sample. That is why in this development concentrations varying from 0.9 nM (nanomolar) of the SNP or STR polymorphic fragments are used, which is the detection limit of Raman SERS; thereby creating an extra level of security since a potential forger must also know the concentration of the polymorphic fragments, to exactly reproduce the same spectrum emitted by the marked item.

The detected values of the Raman spectrum emitted by the polymorphic fragments can be sent via a telecommunication system, an analog telephone line, a digital phone line, cell phone and / or computer connected to a data network, etc.

Once detected and authenticated the marked object, we proceed to the identification of polymorphic STRs or SNPs fragments, typifying them by means of Polymerase chain reaction. In this instance, it is absolutely necessary to know their ID name in order to use the specific reagents and amplification conditions suitable for the corresponding analysis.

It is therefore important to mention that inventors linked each fragment used to mark the object, to a number of existing codes in genebanks. Combining polymorphic STRs or SNPs fragments used, a unique code is created, similar to a PIN number, which corresponds to the exact location within the thousands of polymorphic sites in the genomes of different living beings.

The possessor of such a code, is the owner of the marked object.

In case of controversy, the owner of the marked object can reveal which of the fragments corresponds to each PIN numbers, and any laboratory of molecular biology in the world, can confirm its existence independently of who has been the supplier of these fragments.

Thus, in case of legal dispute, the right of all parties are ensured, because a genetic test concerning the identity of the marked object can be performed independently, anywhere in the world and as often as needed.

Once you reveal the name of the polymorphic fragment used, the DNA typing is performed by the method of Polymerase chain reaction, but may also be carried out by methods and techniques that are common in the prior art such as the use of gels as advocated JM Robertson (1994); capillary electrophoresis according Mc. Cord (1993); multiple hybridization detection or multiple capillary given by Y. Wang (1995), using microchips as set Woolley (1996); mass spectrometry according to Becker (1997); etc. And, SNPs can additionally be detected by a single strand conformational analysis as shown by Orita et al (1989); allele specific oligonucleotide as indicated Landeegren et al (1988); Multiple primer extension according Syvanen and others (1990) or by any other technologies such as microchip, mass spectrometry, etc.

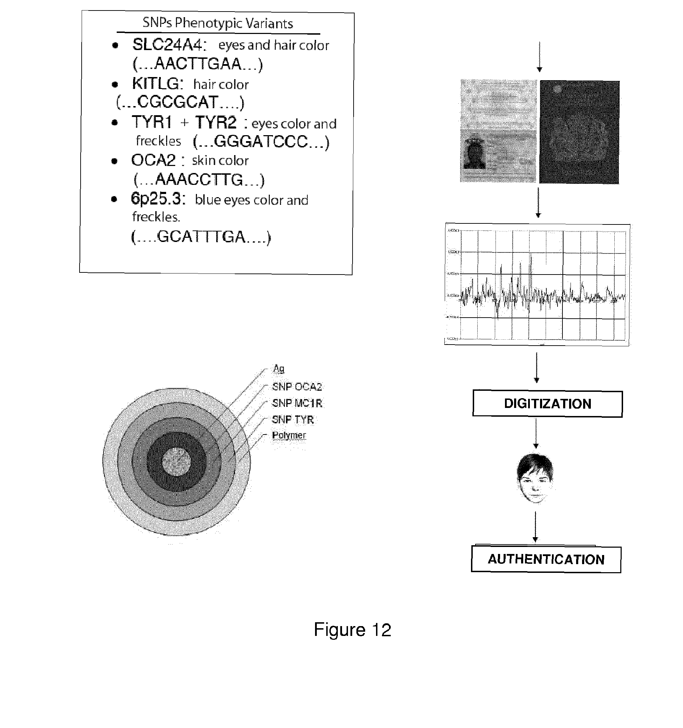

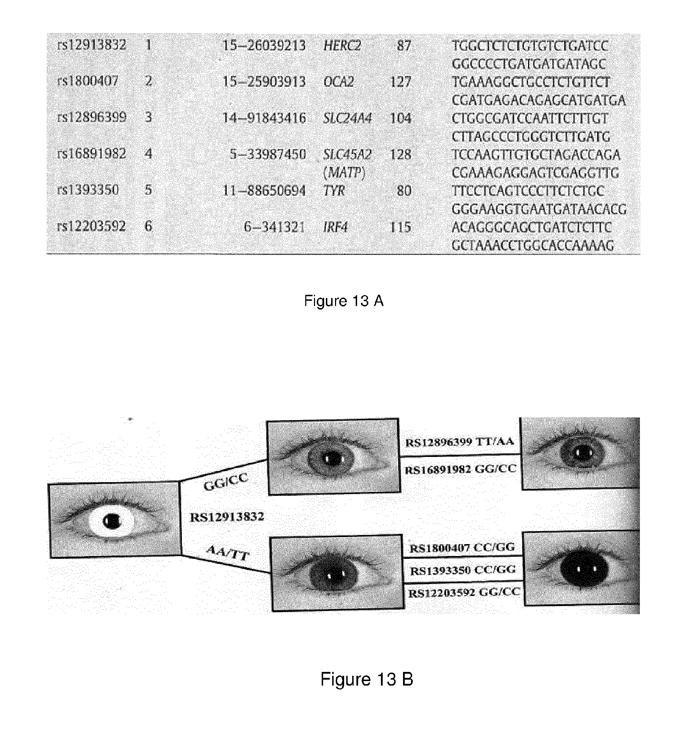

In addition, the inventors know that SNPs are responsible for the phenotypic characteristics of living beings. In the present invention a procedure is included with the same twelve steps above mentioned, where the only polymorphic genetic markers used are SNPs, specially those who are responsible for phenotypic traits that serve to identify the living being or any product derived from them. For example, you can mark any wine with SNPs characterizing color, odor or taste; or a passport, where you can place a drop all fragments of SNPs responsible for the phenotypic characteristics of a person, such as eye color, hair color, skin color, etc. Creating a "genetic identikit", when detected by SERS Raman and digitized with special software; that is possible to check directly with the person who holds the passport. For example variants in the SNP SLC24A4, are associated with the color of eyes and hair, a variant near KITLG is associated with the hair color, two variants of TYR are associated with brown eyes and freckles, a variant 6p 25.3 is associated with freckles, blue eyes color was found in three variants OCA2 SNPs and different skin color tones are related to the 5 'proximal regulatory control OCA2. Several authors are investigating and finding more and more number of related phenotypic characters, which we can mention Sulen, P, Gudbjartsson in genes "Genetic determinants of hair, eye and skin pigmentation in Europeans" Nat Gen 2007 Dec. 39 (12): 1415, and Duffy DL in "A three SNP haplotype in intron 1 of OCA2 Explains MOST human eye color variation" among others; as represented in Figure 12 and 13A and 13B.

With the genetic marker reported in this document, we can marked and identified with absolute certainty innumerable objects such as paintings, sculptures, inputs of sport, art, crafts, video cassette recorders, televisions and any household object, also computers, printers, software, elements of office, and business equipment.

It also may identify perfumes, clothes, handbags, briefcases, boxes of different products, medicine blisters, drugs, parts of automobiles, airplanes, bicycles, stock certificates, tickets, baggage claim tickets, checks, negotiable instruments, commercial papers, legal documents, wills, deeds, contracts, trusts, leases, assignments, easements, postal documents, stamps, bonds, identification cards, birth certificates, driver's licenses, shipping invoices, labels, medical forms, medical records, prescriptions, original art, valuable stamps, bank documents, credit cards, credit card authorizations, invoices, bills, permits, authorizations, applications, and tax returns, bills, currency, checks, documents notary, identity cards, driving licenses, passports, visas, credit cards, telephones and similar objects such as diplomas, inventories, lottery tickets and other games of chance, etc.

The present invention provides various technical complexities to prevent counterfeiting of the marked objects. They consist in the following security levels:
**First Level:** Consists in determining the chemical structure of the polymer. For a counterfeiter to be able to analyze the composition of the polymorphic fragments used to mark the object, he should in the first instance figure out the structure of the polymer used to microencapsulate said fragments, in order to achieve the opening without altering the inside DNA.
**Second Level:** Consist in the identification of the polymorphic fragment. If the forger eventually passes the first level, in order to identify the polymorphic fragments used to mark the object, he must know the name of the polymorphic STRs or SNPs fragments to perform PCR reaction with specific primer pairs and reaction conditions suitable to achieve amplification.
   While one might think that cloning method could be used to identify the polymorphic fragments, but this is not possible; because on the one hand, these polymorphic fragments have been modified and, moreover, the concentrations used in the present development exclude that possibility.
**Third Level:** This involves the characterization of polymorphic fragments, since assuming the forger has managed to evade the above levels must also know the allelic variants of each polymorphic fragments to accurately reproduce those used to mark the object.
**Fourth Level:** consists in working with a concentration of markers that is consistent with the lower limit of polymorphic STR or SNPs fragments used to preclude the use of molecular cloning technique.
**Fifth Level:** This involves the modification of the three-dimensional structure of the DNA molecule using one or a combination of three-dimensional conformations of the molecule, preventing the forger reverse the process if not aware of said modification.
**Sixth Level:** Masking of the markers with additional DNA fragments, so that the forger must know which of the fragments identified by him have been used to mark the object. Consist in the addition of extra DNA fragments to the real ones used to create that unique code number.
**Seventh Level:** The resulting Raman spectrum, forces the forger to know which are the wavelength peaks corresponding to the polymorphic DNA fragments whose three-dimensional structure of the DNA molecule have been previously modified.
**Eighth Level:** Masking of the Raman spectra peaks of the markers, with different Raman active substances. Even if the forger applies SERS methodology, he must distinguish which are the peaks corresponding to polymorphic DNA fragments used to generate the unique code or PIN, and which are the peaks corresponding to the Raman active chemicals that are added to mask the marker.
**Ninth Level:** Consists in coding or encrypting database. If forger surpassed previous secure levels, he must decode to properly interpret and apply the response spectra.
**Tenth Level:** This involves the variation of the relative concentration of each polymorphic fragment, so that authentication is performed by comparing the top and bottom values of the intensity of the peaks obtained in the spectrum emitted by the polymorphic DNA, to the limit values previously stored in a database of response spectra. So, the forger should detect which is the relative concentration of each polymorphic DNA fragment used in markers.

### APPLICATION EXAMPLES

Said description is completed with several examples that have been put into practice, which prompts a whole, with exemplary purposes they serve a purely demonstrative function, but in no case limiting the invention.

Thus it has been reported a possible sequence of steps leading to realize the invention and how it functions, and the documentation is complemented with the synthesis of the invention contained in claiming ownership clauses then added.

### Example #1. The use of DNA inter-species as antifalsification Tag

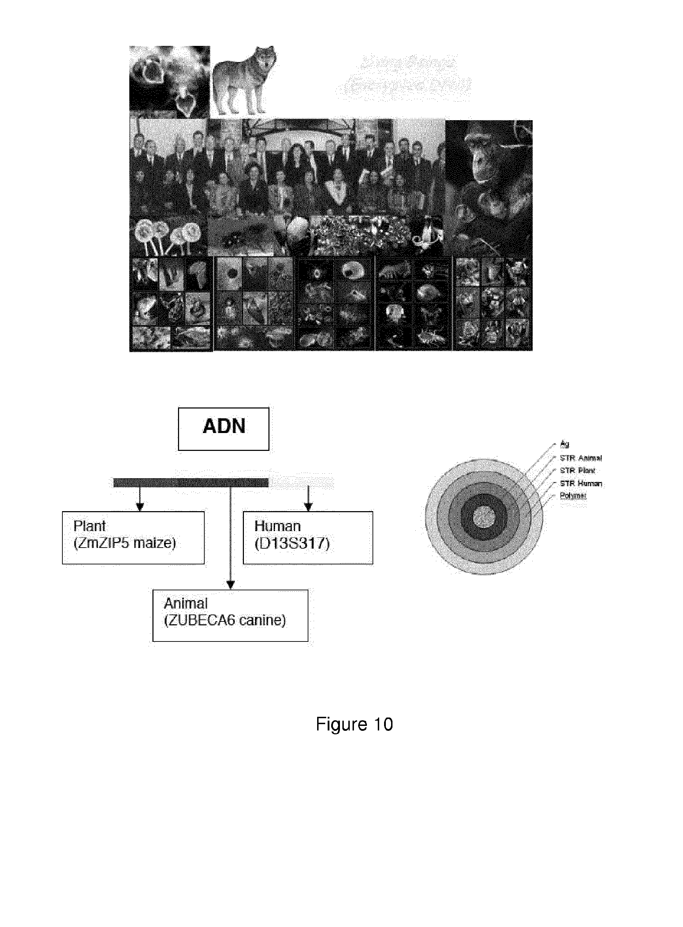

A DNA molecule which does not exist in nature is created. 6 pg of DNA is extracted, and corn gene ZmZ1P5, human gene D13S317, and canine gene ZUBECA6 are amplified; the allelic variants of each locus was determined using ABI PRISM 310 sequencer Applied Biosystem. The final PCR product is mixed with a final solution of 0.25 M silver atoms according to the technique described by Lee. Subsequently the DNA fragments are micro encapsulated in polystyrene, and dissolved in enough water, for accurate application of the marker to the desired object. Raman SERS detection is performed using a DeltaNu Raman Inspector with laser 120 mW at 785 nm, a resolution of 8 cm⁻¹ spectral range 200-2000cm⁻¹. And to authenticate, the data was compared to database with NuSpec data acquisition and library software. In case of litigation, any specialized laboratory in the world can identify the genetic profiles used as tags. But, the microspheres will have to be dissolved with a suitable organic solvent, and once revealed the PIN formed with the access codes to each species gene bank (ex. ZmZIP5ZUBECA6D13S317) the appropriate reagents (primers) may be used, to analyze the allelic variants by PCR (see Figure 10).

### Example #2. The use of personal DNA as antifalsification Tag

Personal DNA molecule is created. In case of litigation, PIN with the access codes of gene banks of each STR/SNP is revealed; as well as the allelic variants of each of the fragments used (ex. X0629288X147201011M8652545), and any specialized laboratory in the world can recreate the genetic profiles without depending on the manufacturer of the products.

### Example #3. The use of SNPs (phenotypic traits) for passports authentication

The use of SNPs responsible for the phenotypic characteristics of a person, like eye color, hair, skin color, etc., could create a "genetic identikit" when detected by SERS Raman and digitized; it allows direct comparison with the person who owns the passport.

0.5 ugr DNA from saliva of a person is extracted and the six SNPs genes that are detailed below (in Figure 13A) are analyzed. The possible hypothetical scenarios for determining brown or blue color eyes, according to genotypic variants are amplified of these six SNPs, as shown in Figure 13B.

### Example #4. The use of Microspheres containing polymorphic DNA as antifalsification Tag in paper money

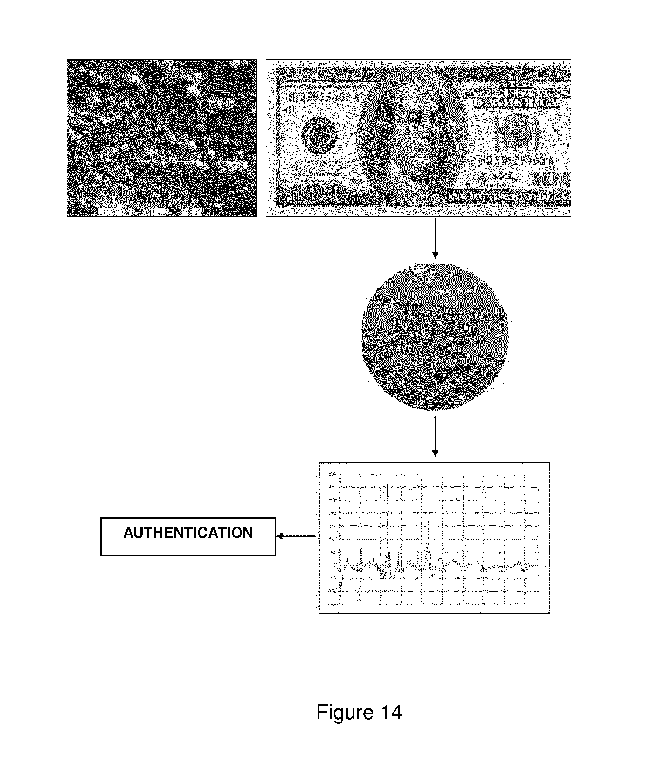

The use for this purpose, is represented in Figure 14.

## Claims

**1.** Procedure for obtaining a marker of objects to be identified, comprising at least a first step of selecting a living being for DNA extraction, and an eighth step of determining and correcting the degree of fluidity of the solution; comprising also;
• a second step, of including the obtained sample in a solution containing between 9 and 10.2 mM Tris-HCl; between 0.95 and 0.11 EDTA; 20% SDS (w/v) and from 9.8 to 10.3 mg/ml Proteinase K, proceeding to the purification with phenol/chloroform at a ratio of 10: 9 (v/v);
• a third step, of amplifying short tandem polymorphic fragments (STRs) or single nucleotide polymorphisms (SNPs) present in the DNA sample;
• fourth step, of determining the allelic variants of polymorphic fragments chosen;
• a fifth step, of modifying the three dimensional structure of polymorphic STRs/SNPs fragments and adsorption to nanoparticles of at least one metal;
• a sixth step, of concentration and microencapsulation of polymorphic fragments; and
• a seventh step, of solubilization of the DNA microspheres or microencapsulated DNA to be detected by Raman spectroscopy.

**2.** Procedure according to claim 1, because in the fifth step, said metal is selected from gold, silver, platinum or copper.

**3.** Procedure according to claim 1, because in the fifth step the three-dimensional structure of DNA is modified by a method selected from:
I. individual added or as colloids of metal nanoparticles with DNA adsorbed which produces aggregates as dimers, trimers, or tetramers with the DNA molecules; at least a liking group is added to metal nanoparticles selected from a polymer, a silane or an alkane and its derivatives, and which the polymer is selected from phenylacetylene, polytetrafluoroethylene, polypropylene, polyacrylamide or similar.
II. the added DNA complexes - dendrimers PAMAM type or similar, to form a characteristic three-dimensional structure with a single Raman spectrum,
III. by the covalent attachment of polymorphic DNA fragments with synthetic type PNAs (peptide nucleic acid),
IV. by binding fragments of polymorphic STRs/SNPs with aptamers obtained with the SELEX process,
V. by twisting of the DNA double helix, by using intercalating molecules between bases, and along said double helix; intercalating agents which are selected from ethidium bromide, adriamicyn, 9-aminoacridine (9AA) and proflavine (PF) (3,6-diaminoacridine),
VI. by precipitation or aggregation of a protein to the major and minor grooves of DNA, using a drug selected from Spermine, spermidine, putrescine, Hoechst 33258, Netropsin, Pentamidine, or similar,
VII. by aggregate formation, which react with phosphate and/or nitrogenous bases, destabilizing the double helix by breaking DNA hydrogen bonds, using divalent metal complexes such as Sr⁺², Ba⁺², Mg⁺², Ca⁺², Mn⁺², Co⁺², Ni⁺² and Cd⁺².

**4.** Procedure according to claim 3, because in said item VI, the interaction of these drugs with the major and minor grooves of DNA is directly related to the amount of AT, hence the sequence of each fraction STR or SNP can obtain a characteristic spectrum of polymorphic fragment used to mark the object.

**5.** Procedure according to claim 1, in the said fifth step, the metal nanoparticles with adsorbed DNA, incorporated to the marker increase the Raman SERS signal between 10⁶ and 10¹⁴ times.

**6.** Procedure according to claim 3, in the said fifth step, the linking group is selected from silanes and alkanes molecules or derivatives thereof linked to DNA molecules forming aggregates characteristic of a molecular network forming a single Raman spectrographic signal.

**7.** Procedure according to claim 1, in the said sixth step, we proceed to concentrate by ultracentrifugation and to microencapsulate the polymorphic DNA fragments by phase inversion technique; polymorphic DNA is dissolved in a solvent, and then in the same solvent, a polymer is also dissolved with a concentration between 0.25% and 10% w/v; which is selected from biodegradable and non-biodegradable.

**10.** Procedure according to claim 7, in the said sixth step, the non-biodegradable polymer is selected from ethylene vinyl acetate, polyacrylic acid, polyamides, and copolymers and mixtures thereof.

**12.** Procedure according to claim 7, in the said sixth step, the solvent / non-solvent ratio is between 1/40 and 4/200.

**13.** Procedure according to claim 7, in the said sixth step, we proceed to the concentrated by ultracentrifugation with microconcentrators such as Centricon 100.

**14.** Procedure according to claim 1, because the concentrations of polymorphic DNA fragments used are variables from 0.9 nM.

**15.** Marker according to claim 14, because each polymorphic DNA fragment used is assigned a number of existing code in genebanks so that the combination of polymorphic fragments STRs/SNPs used in the marker, creates a unique code number that corresponds to the exact sites within the polymorphic sites in the genome of living organisms, or whose DNA was used.

**16.** Marker according to claim 14, selected polymorphic DNA fragments are masked incorporating other DNA fragments than those chosen to form the unique number.

**17.** Marker according to claim 14, the Raman spectrum of polymorphic DNA fragments is masked, by the addition of Raman active organic substances, without the affection on the original spectrum emitted by the polymorphic fragments.

**18.** Detection method according to claim 1, said method comprises the detection of polymorphic DNA fragments included in said marker with a method selected from the following but not limited to: Normal Raman scattering; Resonance Raman scattering; Surface Enhanced Raman scattering; surface enhanced resonance Raman scattering; coherent anti-Stokes Raman spectroscopy (CARS); stimulated Raman scattering; inverse Raman spectroscopy; stimulated Raman gain spectroscopy; hyper-Raman scattering; molecular optical laser examiner (MOLE) or Raman microprobe or Raman microscopy or confocal Raman microspectrometry; three-dimensional or scanning Raman, NIR spectroscopy, Raman saturation spectroscopy; time resolved resonance Raman; Raman spectroscopy decoupling or UV-Raman microscopy.

**19.** Authentication method according to claim 1, said method comprises a first step of suppressing the characteristic fluorescence of DNA, and a second step of comparing the upper and lower values of the intensity of the peaks obtained in the spectrum emitted by the polymorphic DNA fragments with limit values previously stored as reference; and give an instant authentication response, positive or negative match.

**20.** Authentication method according to claim 19, in the said first step of elimination of the fluorescence, comprising a step of determining an average intensity value in the spectral data obtained within a section around each point of said response spectrum and a step of subtracting said medium value to each of the points of the DNA fragments used.

**21.** Authentication method according to claim 19, in the said second step comprises a step of comparing the Raman peak data obtained with the Raman peak data stored in a database, and a following step of comparison of the wavelength numbers and intensity of each peak, with the spectrographic data stored in database.

**22.** Authentication method according to claim 19, said data stored corresponds to the spectrums of each of the polymorphic STRs/SNPs fragments used to mark the object.

**23.** Authentication method according to claim 19, the detected values of the Raman spectrum emitted by the polymorphic DNA fragments are sent through any of the following telecommunication systems: an analog telephone line; a digital phone line; a cell phone; a computer connected to a data network or equivalent.

**24.** Verification method according to claim 1, once detected and authenticated the marked object, it proceeds to identify fragments of polymorphic STRs/SNPs through Polymerase chain reaction.

**25.** Verification method according to claim 24, comprises a step of establishing the name of polymorphic fragments STRs/ SNPs and a step of adapting the reagents to appropriate amplification conditions.

**26.** Verification method according to claim 24, the number assigned to a single polymorphic fragment and included in a database can be determined by analysis of the polymorphic fragment by molecular biology techniques and comparing the results of the analysis with the evidence in this database.

**27.** Verification method according to claim 24, typing or polymorphic fragments STRs and SNPs is performed with procedures and techniques that are common in the prior art such as the use of gels; capillary electrophoresis; multiple hybridization detection or multiple capillary; using microchips and mass spectrometry and others.

**28.** Verification method according to claim 24, detection of single base polymorphisms is performed by a single strand conformational analysis; or allele specific oligonucleotide; by multiple primer extension or by any other technology that may be mentioned among the chips and mass spectrometry.

**29.** Method to incorporate the marker to the object to be identified, where the marker is obtained according to claim 1, said method comprises a step of incorporating said label to a fluid, and a step of including the fluid containing said marker to an applicator, wherein the marker contained in the solution is present in a concentration of between 6 and 10 pg per mm² of surface.

**30.** Method to incorporate the marker to the object to be identified according to claim 29, microspheres with polymorphic DNA fragments are solubilized in different varieties of ink, such as flexographic inks, lithographic inks, screen inks, gravure inks, reactive currency inks, erasable inks, pen reactive ink, heat reaction inks, inks visible to infrared, optically variable inks, penetrating inks, photochromic inks, chemical reactive to solvents or water.

**31.** Method to incorporate the marker to the object to be identified according to claim 29, the applicator is selected from a pen to pen, microfiber, a pen, an atomizer, a tool for drawing, a brush, a stamp, an electrophotographic printer type inkjet, an offset lithography or letterpress, gravure, of xerography, screen printing, a system of textile printing or similar.

**32.** Method to incorporate the marker to the object to be identified according to claim 29, between the solution containing the marker and the object to be marked, a means intermediary embedded in said solution is employed, and selected from nitrocellulose, paper, wood, cardboard, plastic, reinforced nylon, cloth, organic substances as droplets or inorganic gel.

**33.** Method to incorporate the marker to the object to be identified according to claim 29, the solution containing the label is incorporated into paintings, sculptures, sports supplies, artwork, crafts, video cassette recorders, televisions and any household object, also computers, printers, software, office items and equipment business, perfumes, clothes, handbags, briefcases, boxes of different products, blister medicines, drugs, parts of automobiles, airplanes, bicycles, stock certificates, tickets, baggage claim tickets, checks, negotiable instruments, commercial papers, legal documents, wills, deeds, contracts, trusts, leases, assignments, easements, postal documents, stamps, bonds, identification cards, birth certificates, driver's licenses, shipping invoices, labels, medical forms, medical records, prescriptions, works original art, valuable stamps, bank documents, credit cards, credit card authorizations, invoices, bills, permits, authorizations, applications, and tax returns, bills, currency, checks, legal documents, identification cards, licenses driving, passports, visas, credit cards, telephones and similar objects such as diplomas, inventories, lottery tickets and gambling.

**34.** Procedure according to claim 1, comprises the following levels: a first level which consists in determining the chemical structure of the polymer used to microencapsulate the polymorphic DNA fragments; a second level which involves the identification of the modified polymorphic DNA fragments used; a third level consisting in typing or identifying allelic variants of each of the polymorphic fragments; fourth level consist in adapting the concentration of markers to the lower limit of polymorphic STRs/SNPs fragments used; a fifth level that is the modification of the three dimensional structure of the DNA molecule using one or a combination of three-dimensional conformations of the said molecule; a sixth level, consisting in masking markers with the addition of different DNA fragments; a seventh level, a seventh level, which consists in using the Raman spectrum of polymorphic fragments modified; an eighth level, which consists in masking the Raman spectra of the markers with different active substances Raman spectra; a ninth level, consisting in coding the database; and a tenth level, that is to vary the relative concentration of each polymorphic fragment.
